Europäisches Patentamt

European Patent Office

Office européen des brevets

(19)

(11) Publication number: 0 356 409
A2

(12) **EUROPEAN PATENT APPLICATION**

(21) Application number: 89850249.7

(22) Date of filing: 08.08.89

(51) Int. Cl.⁵: **C 12 P 21/02**
C 12 N 15/62, C 12 N 15/12,
C 12 N 15/85, C 12 N 15/86,
C 07 K 3/02, C 12 P 21/08

(30) Priority: 19.08.88 SE 8802939

(43) Date of publication of application:
28.02.90 Bulletin 90/09

(84) Designated Contracting States:
BE CH DE FR GB IT LI NL SE

(71) Applicant: KabiGen AB
S-112 87 Stockholm (SE)

(72) Inventor: Hansson, Lennart
Killingevägen 24B
S-181 64 Lidingö (SE)

Josephson, Staffan
Högstigen 2
S-182 74 Stocksund (SE)

Steiner, Hakan
Tulpanvägen 9
S-186 00 Vallentuna (SE)

(74) Representative: Henningsson, Gunnar et al
Bergling & Sundbergh AB Box 7645
S-103 94 Stockholm (SE)

(54) **A method for the production and isolation of a fusion protein in eukaryotic cells.**

(57) A method for the production and isolation of a fusion protein, comprising the steps:

a) constructing a recombinant vector comprising a first DNA sequence coding for a desired peptide or protein and operatively linked to a second DNA sequence coding for an amino acid sequence capable of selective binding to a carrier material, said first or second DNA sequence being preceded by and being operatively linked to a third DNA sequence coding for a eucaryotic signal peptide;

b) transforming a compatible eucaryotic host with the recombinant vector resulting from step a) such that the combined DNA sequences coding for said fusion protein can be expressed and secreted by said host;

c) culturing said host to produce said fusion protein;

d) isolating the fusion protein resulting from step c) by adsorption to a carrier material; and

e) desorbing said fusion protein from said carrier or removing by cleavage said peptide or protein from said amino acid sequence adsorbed on said carrier; and

f) recovering said fusion protein or said peptide or protein;

recombinant nucleotide sequence;

recombinant vector containing such nucleotide sequence;

a process for preparing polyclonal antibodies using a fusion protein; and

the use of the fusion protein as a vaccine or for the manufacture of monoclonal antibodies.

EP 0 356 409 A2

## Description

### A method for the production and isolation of a fusion protein in eukaryotic cells.

The present invention relates to methods for expressing an exogenous gene in a selected eukaryotic cell and products thereof. More particularly, this invention relates to a method for production of a desired protein fused to another peptide sequence which facilitates isolation and purification of the desired product in combination with appropriate signals for secretion to extracellular medium. The invention also includes a recombinant nucleotide sequence for use in the method, a recombinant vector containing such recombinant nucleotide sequence, a eukaryotic cell harbouring such nucleotide sequence or vector, and the use of the fusion protein prepared by the method as a vaccine or for the manufacture of monoclonal antibodies.

Another aspect of this invention relates to the expression of peptides in eukaryotic systems which allow correct folding and posttranslational modification in combination with a fusion partner which facilitates purification and appropriate signals for extracellular distribution as well as selected cleavage of the fusion protein and isolation of the desired product. In accordance with the present invention a DNA fragment encoding a desired amino acid sequence is isolated, synthesized or otherwise obtained. The so obtained DNA fragment is inserted into an appropriate expression vector in such a way that it becomes joined in phase to a DNA part encoding the signal sequence, the carrier protein as well as other regulatory sequences needed for high level expression in a host cell.

The recombinant DNA technology has provided methods for production of different macromolecules by using different strategies.

For several reasons e.g. purification, production, vaccine production, generation of peptide specific antibodies, the synthesis of fusion proteins are of great interest. Some strategies have been reported for such production in prokaryotic organisms (Löwenadler et al. 1987). The gene fusion techniques have been used to monitor the expression from a gene, to allow secretion or to facilitate the downstream processing. This method also provides a system for isolation of a cleavage product, a part of the fusion protein, by either chemical or enzymatic cleavage.

The lack of enzymatic activity providing posttranslational functions such as glycosylation etc. in prokaryotic expression systems seriously limits the use of such systems for production of gene fusion proteins with native structure.

The principal object of the present invention is to eliminate or at least greatly reduce the deficiencies associated with the conventional art. Other objects and aspects of the invention will be clear from the following disclosure.

Accordingly, the present invention provides a method for the production and isolation of a fusion protein. The present method comprises the following steps:

a) constructing a recombinant vector comprising a first DNA sequence coding for a desired peptide or protein and operatively linked to a second DNA sequence coding for an amino acid sequence capable of selective binding to a carrier material, said first or second DNA sequence being preceded by and being operatively linked to a third DNA sequence coding for a eucaryotic signal peptide;

b) transforming a compatible eucaryotic host with the recombinant vector resulting from step a) such that the combined DNA sequences coding for said fusion protein can be expressed and secreted by said host;

c) culturing said host to produce said fusion protein;

d) isolating the fusion protein resulting from step c) by adsorption to a carrier material; and

e) desorbing said fusion protein from said carrier or removing by cleavage said peptide or protein from said amino acid sequence adsorbed on said carrier; and

f) recovering said fusion protein or said peptide or protein.

It is preferred in such method that said amino acid sequence capable of selective binding is of an immunogenic character. Using such amino acid sequence in the fusion protein of the present invention results in an improved antibody generation when the fusion protein is used for immunization.

It is particularly preferred to use as an amino acid sequence one that is selected from sequences capable of binding to the constant regions of immunoglobulins such as the IgG-binding region of protein A. The host used in the method of this invention is suitably selected from insect and mammalian cells.

The invention also provides for a recombinant nucleotide sequence comprising a DNA sequence coding for a desired peptide or protein and operatively linked to a second DNA sequence coding for an amino acid sequence capable of selective binding to a carrier material, said first or second DNA sequence being proceeded by and being operatively linked to a third DNA sequence coding for a eucaryotic signal peptide. In such nucleotide sequence said second DNA sequence preferably codes for an immunogenic amino acid sequence. Capability of binding to the constant regions of immunoglobulins, such as the IgG-binding region of protein A, is preferred.

The invention also provides for a recombinant vector comprising a first DNA sequence coding for a desired peptide or protein and operatively linked to a second DNA sequence coding for an amino acid sequence capable of selective binding to a carrier material, said first or second DNA sequence being proceeded by and being operatively linked to a third DNA sequence coding for a eucaryotic signal peptide.

Within the scope of the invention there are also eukaryotic cells harbouring the recombinant nucleotide sequence or the recombinant vector as outlined above.

Furthermore, the invention provides for a process for preparing polyclonal antibodies, such process comprising immunizing an animal using the fused protein as defined above, then bleeding the animal and recovering the generated antibodies from the animal serum. The animal can be selected from mammals, such as rabbits or mice. The fusion protein may also be used for the manufacture of monoclonal antibodies or as a vaccine.

The present invention thus sets out to provide a process whereby fusion proteins can be produced and secreted in extracellular medium by eukaryotic cells thereby providing a system for correct post-translational modification as well as folding etc. More specifically the invention deals with the production of peptides whose activity is dependent of eukaryotic processing functions, production of antibodies against a desired amino acid sequence be it a protein, a peptide or a short stretch of acids.

The gene fusion system described in the present invention provides a DNA segment encoding an appropriate eukaryotic signal peptide, followed by a sequence encoding an amino acid sequence characterized by specific binding properties, combined with a sequence encoding any desired peptide or protein. The preferred amino acid sequence with specific binding properties can be structures with affinity for: a) the constant regions of immunoglobulin such as Staphylococcus aureus Protein A or derivatives thereof; b) carbohydrates such as lectins or enzymes as RNase; c) fibrin structures such as the kringle structures derived from tissue plasminogen activator or plasminogen; d) biotin such as avidin; e) cation exchangers such as polylysine; f) gelatin such as fibronectin structures; g) DNA such as fibronectin structures or chromatin proteins; h) antibodies such as antigens. The desired peptide or protein encoding sequence fused in phase to the described amino acid encoding sequence for expression in eukaryotic hosts can preferentially encode for peptides or proteins which require post-translational modifications such as glycosylation, carboxylation, amidation etc and/or require adequate folding. Examples of such peptides or proteins are: a) peptide hormones e.g. gastrointestinal peptides as secretin, vasoactive peptide etc or neuropeptides as nerve growth factor (NGF), brain insulin like growth factor (brain-IGF) etc or other growth factors such as IGFI, IGFII, TGF, FGF, EGF, PDGF, IL-2 and others; b) antibacterial peptides e.g. cecropins, magainins etc; c) subunit vaccines e.g. HIV-gp 120 etc; d) toxins e.g. melittin; or any other peptides or proteins of interest.

By expression of a carrier protein gene construct in combination with a DNA segment encoding a peptide sequence in a suitable eukaryotic host cell system, naturally occuring posttranslational modifications in that peptide sequence can be obtained. A peptide sequence comprising a neutralization site of a pathogen (e.g. virus, bacteria etc) dependent partially or fully on structures obtained by posttranslational modifications can thus be produced.

Antibodies may be generated by immunizing appropriate animals with the fusion protein, containing the correct modified structure. The fusion partner, the carrier protein, provides facilitated recognition of the desired peptide by the immune system of the inoculated organisms. Using a carrier molecule with polyclonal B-cell stimulatory effect may further enhance the immune response in the immunized hosts.

A further aspect of the invention relates to the use of gen-fusion peptides expressed in eukaryotic cells for obtaining specific immune responses. More specifically one aspect relates to the production of recombinant DNA derived vaccines.

Selected positions of a pathogen's genome, those coding for structural antigenic sites involved in the induction of a protective immune response against the pathogen, may be expressed in bacterial or eukaryotic cells, and used to produce neutralizing antibodies in immunized animals. This was first shown for the virus of foot and mouth disease (Kleid et al. 1981).

Important antigenic sites in a protein may consist of linear or conformational peptide structures as well as structures obtained by posttranslational modifications (e.g. glycosylation, amidation, carboxylation etc.) or combinations thereof.

For many pathogens protein epitopes comprising structures obtained by prosttranslational modifications may be vital for raising neutralizing antibodies or for inducing cell mediated immunity.

In development of a vaccin against an infectious agent several structures may be of importance for the complex immunological reactions leading to protective immunity.

Such structures are e.g. T-cell helper inducing determinants, T cytolytic inducing determinants, B cell inducing determinants and T suppressor inducing determinants. By dissecting regions of a pathogens genome encoding such determinants and co-expressing them, using a single gene construct, a fusion protein harboring several immunologically important regions can be produced.

Plasmid DNA, designated pKGE-132, has been deposited in the collection of: "Deutsche Sammlung von Mikroorganismen und Zellkulturen GmbH, Mascheroder Weg 16, D-3300 Braunschweig, Federal Republic of Germany" on August 10, 1988 and has been identified there by accession number DSM 4758.

In the following the present invention will be further illustrated by non-limiting examples. This exemplification will be made with reference to the annexed figures.

BRIEF DESCRIPTION OF THE ANNEXED FIGURES

Fig. 1 This figure describes the construction of the vector pKGE-91. The vector pKGE-91, is a baculovirus transfer vector based on pAC-610 and with human t-PA cDNA included.

Fig. 2 This figure is a schematic representation of the ZZ fragment and also the ZZ sequence is listed.

Fig. 3 The sequence of the synthetic cecropin A encoding fragment.

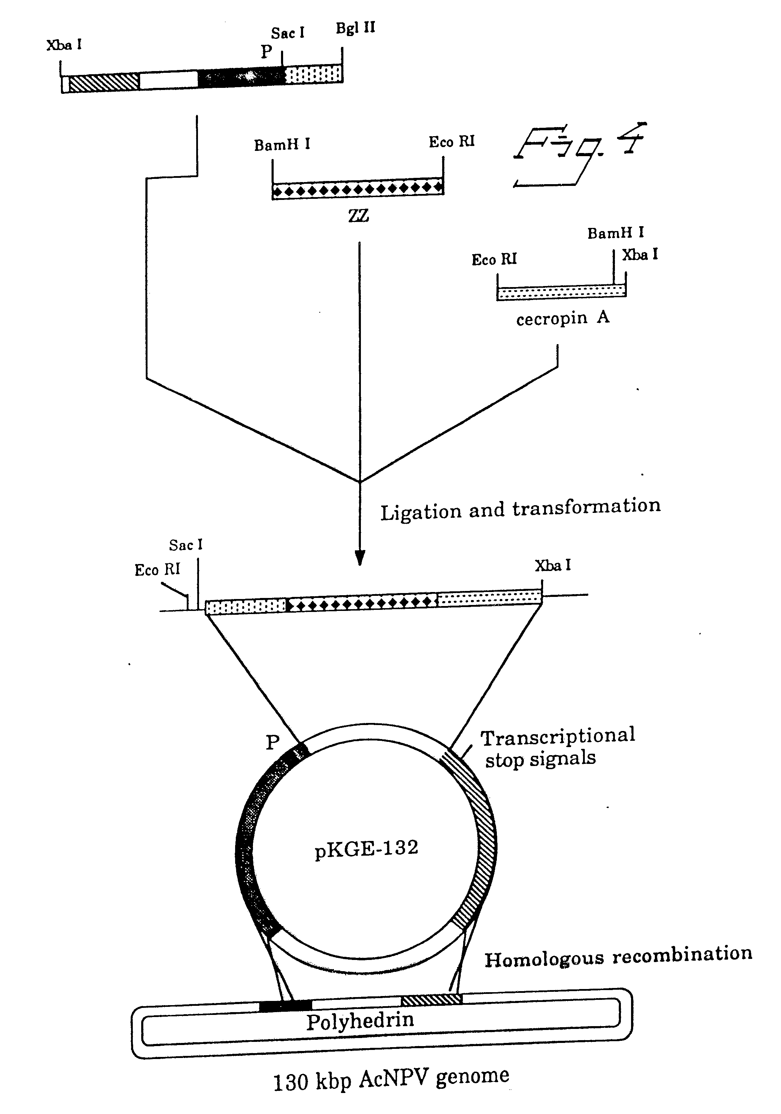

Fig. 4 This figure illustrates the ligation of the three fragments; polyhedrin-tPA signal peptide, encoding fragment, the ZZ encoding fragment and the cecropin A encoding fragment. The resulting transfer vector is also schematically represented as well as the construction of recombinant baculovirus, by introduction of sequences by homologous recombination into baculovirus genome.

Fig. 5 shows SDS-PAGE and Western blot of IgG affinity purified ZZ-cecropin A and cleavage with CNBr.

Fig. 6 shows antibacterial activity of recombinant cecropin A on an inhibition zone agarose plate with E.coli D21.

Fig. 7 shows the mobility of recombinant cecropin A and natural cecropin A on acidic gel electrophoresis with antibacterial detection.

Routine Methods

The routine methods used in accordance with the cloning procedures set forth in the examples are described in T. Maniatis, E.F. Fritsch and J. Sambrook, Molecular Cloning: A Laboratory Manual, Cold Spring Harbor Laboratory, 1982. This reference includes procedures with E.coli plasmids, transformation of E.coli cells, plasmid DNA purification, phenolextraction of DNA, ethanol precipitation of DNA, agarose gel electrophoresis, isolation of DNA fragments from agarose gels, and restriction endonuclease and other DNA-modifying enzyme reactions.

Enzymes were bought from Amersham, NEN, or Boehringer Mannheim.

The procedures described in: Summers M.D. and Smith G.E.: A manual of methods for baculovirus vectors and insect cell culture procedures. Tex.Agric.Exp.Stn.Bull. (1987) No 1555, were followed for all baculovirus vector and insect cell work.

EXAMPLE 1

Construction of expression vectors

This example describes the isolation, synthesis, characterisation as well as the assembly of DNA fragments in order to obtain an expression vector.

Isolation of the t-PA fragment encoding the signal peptide

Bowes melanoma cells, a transformed human cell line which constitutively produce high levels of t-PA, were cultured in glass roller bottles at +37°C in Eagles minimal essential medium (EMEM) supplemented with 1% non essential amino acids (Flow), glutamine (2mM), penicillin (50 IU/ml), streptomycin (50 µg/ml), Hepes (20 mM, pH 7.2) and 10% foetal calf serum.

Confluent cultures of melanoma cells were harvested by trypsination. After washing in ice cold phosphate buffered saline pH 7.2, the cells were recovered by centrifugation. Cell pellets were lysed in 4M GuSCN, and total RNA was then selectively precipitated from a GuHCl solution by ethanol. Messenger RNA was purified from the total RNA preparation by chromatography on oligo-dT cellulose. The poly-A+ mRNA obtained was size fractioned on a sucrose gradient, consisting of 10-30% sucrose in 50 mM LiCl, 20 mM Tris-HCl, 1mM EDTA-Li, 1% LiDS and with a pH of 7.8.

Sucrose fractions corresponding to 23 S were shown to be enriched in t-PA mRNA by dot blot hybridization. 5µg mRNA from this enriched fraction was used to construct a cDNA library essentially as described by Okayama & Berg (1983) with the modification detailed in Hedén et al. (1986). The cDNA containing plasmid pT4 prepared by the disclosed method was used to transform E.coli5K. The gene bank obtained consisted of approximately 5x10⁴ independent clones.

The isolated partial t-PA cDNA clone (Edlund et al. 1983) was used to screen the gene library for t-PA sequence containing clones. A 476 bp RI fragment of the cDNA clone was nicktranslated with ³²P-dCTP to a specific activity of about 5x10⁷CPM/µg using a commercial nicktranslation kit (NEN). Bacterial colonies were transferred to PALL filters by the method suggested by the manufacturer and were hybridized to the nicktranslated DNA probe in 50% formamide, 5xSSC, 250 µg/ml yeast RNA at 37° over night. After washing in 3 changes of 2xSSC at ambient temperature, the filters were dried and exposed on X-ray film.

One clone, designated pKG12, was identified after autoradiography. Digestion with the restriction enzymes Kpn I and Pst showed that pKG12 contained an insert of about 2.5 kb. The insert was subcloned into M13 mp10 and pm11 (Messing 1983) and DNA sequence analysis was performed using the dideoxy chain termination method (Sanger et al., 1977). Clone pKG12 was shown to comprise the whole coding region for human t-PA as well as 102 bp 5' flanking, 760 bp 3' flanking DNA and a poly A tract.

To obtain the fragment encoding tPA and all of the 5'noncoding region and 559bp of the 3' 19 noncoding region, 25 µg pKG12 was cleaved to completion with 30 units Xmn I overnight at 37°C. Ava I (9 units) was added and the cleavage continued for another 1h and 20 min at 37°C. The various cleavage products obtained were separated in a 0.6% low gelling temperature agarose gel (LGT-agarose, Bio-Rad).

The 2350 bp band was excised and melted at 68°C, followed by phenol extraction and ethanol precipitation. The purified fragment was made blunt ended by a fill-in reaction using the Klenow fragment of DNA polymerase in the presence of dNTP:s. Bam HI linkers (5'-CGCGGATCCGCG-3') were added to the fragment using T4 DNA ligase. After subsequent cleavage with Bam HI and removal of excess Bam HI linker-fragments by 0.7% LGTagarose electrophoresis, the t-PA fragment was ligated to Bam HI cleaved pUC8.

The resulting plasmid, denoted pKGE22 contains most of the t-PA cDNA within a Hind III/Bam HI fragment. The sequence downstream of the Xmn I site, harboring the putative polyadenylation signal AATAAA has been removed.

The ligation mixture was used to transform competent E.coli JM83 cells. White colonies were selected on agar plates containing 50µg/ml ampicillin and 25µl/plate of 2% Xgal in dimethylformamide.

## Construction of a sequence encoding an IgG binding domain

The desired DNA sequence was analysed by a computer program and divided into 10 oligonucleotides varying in length from 41 to 45 nucleotides and with an overlap of 6 bp.

Synthesis was effected on a fully automated machine and the deprotected oligomers were purified by polyacrylamide electrophoresis (20% polyacrylamide, 7 M Urea, 50 mM Trisborate pH 8.3) followed by extraction into water and lyophilization.

100 pmoles of oligonucleotides A1-A5 and B1-B5 were phosphorylated separately in 20 μl Kinase buffer (50 mM Tris-HCl pH 7.6, 10 mM MgCl2, 1 mM ATP. 10 mM DTT) 5 units of polynucleotide kinase was added and the mixtures were incubated for 45 minutes at 37°C.

5μg of the vector M13mp18, replicative form, was digested with the restriction enzymes EcoRI and HindIII. The large fragment from the digestion was isolated from a low temperature agarose gel.

The agarose containing digested M13mp18 was melted at 65°C and 5μl (0.5μl, 0.1 pmole) was mixed with 0.5 pmole each of the phosphorylated oligomers A1-A5 and B1-B5 in 50μl ligation buffer (66 mM Tris-HCl pH 7.6 50 mM MgCl2, 50 mM DTT, 1 mM ATP) heated to 90°C and slowly cooled to room temperature during one hour. 10 units of T4 DNA ligase was added and the mixture was incubated overnight at 15°C.

E.coli JM 103 was transfected with the DNA thus obtained and grown overnight on 2xYT plates containing x-gal and IPTG. 78 white plaques were transferred to a new 2xYT plate and grown as colonies. Colony hybridization with $^{32}$P labelled oligonucleotide B5 as probe gave one positive colony which was picked and grown in 15 ml 2xYT with E.coli JM 103. The cells were spun down and the phages were recovered from the supernatant. The single stranded phage DNA was extracted and purified and was used as template for sequencing reaction according to the dideoxy method. The M13mp18 containing the Z-fragment was designated M13-Z.

The construction was confirmed by DNA sequencing.

The single stranded DNA from M13-Z was annealed to sequencing primer followed by treatment with Klenow and dNTPs. In this way double stranded DNA was obtained. The reaction mixture was digested with HindIII and EcoRI and the Z fragment was isolated using a Low Gel Temperature Agarose gel electrophoresis.

Separately the plasmid pUC 8 was digested with EcoRI and HindIII and the large fragment was isolated using Low Gel Temperature Agarose gel electrophoresis. The two isolated gel fragments were heated to 65°C and 2μl of each melted fragment were ligated in 50μl ligation buffer and 2.5 units DNA ligase at 15°C overnight. The DNA thus obtained was used to transform E.coli strain JM83 as above, and plasmid DNA from two transformants was isolated and proved to contain the Z fragment. The pUC 8-Z was digested with restriction enzyme AccI. After religation of the digestion mixture a new transformation of JM83 was made, followed by plasmid isolation from 12 transformants. Digestion with HindIII and EcoRI and analysis on agarose gel confirmed that 2 transformants carried the pUC 8 with an insert of two Z fragments (pUC 8-ZZ).

## Construction of plasmid pKP181A

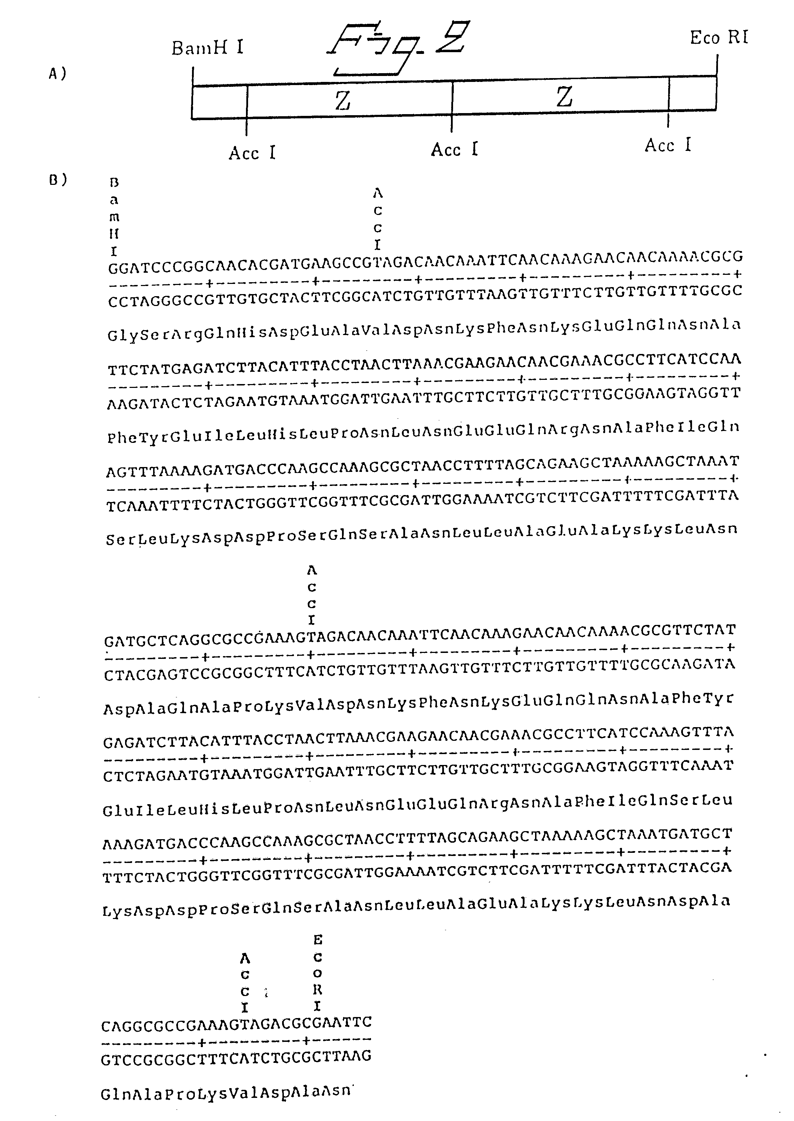

pUC18-ZZ was digested with FspI. A phosphorylated BamHI linker was ligated to the FspI sites. (The BamHI linker had the following sequence 5′-CGGGATCCCG). After ligation the mixture was cleaved with BamHI and EcoRI and the fragment containing the ZZ region was isolated on LGT agarose. This fragment was then subcloned into pUC18 opened at the EcoRI and BamHI sites. The plasmid was designated pKP181 and it has the pUC18 cassette from BamHI to HindIII upstream the ZZ region. See fig 2.

## Synthesis of Cecropin A

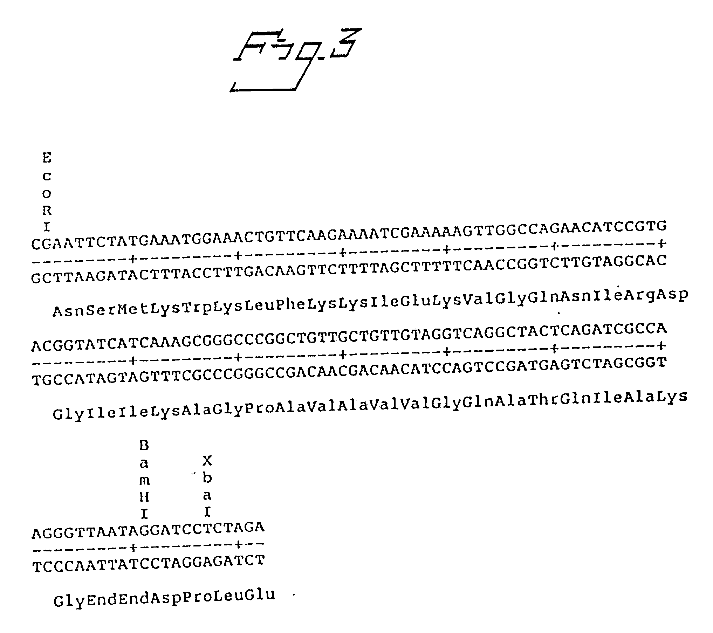

The Cecropin A coding sequence was synthesized by using the phosphoamidite method (Elmblad et al. 1982) Figure 3.

## Introduction of the fragments into the transfer vector pAC-610

pKGE-22 was cleaved at the unique HindIII site and a SacI linker was added by standard procedures. After ligation, transformation and preparation of the modified pKGE-22 the tPA encoding fragment was isolated by partial SacI and BamHI digestion and low temperature melting agarose electrophoresis. The baculovirus transfer vector pAc-610, described in Luckow and Summers (1988), was digested with SacI and BamHI.

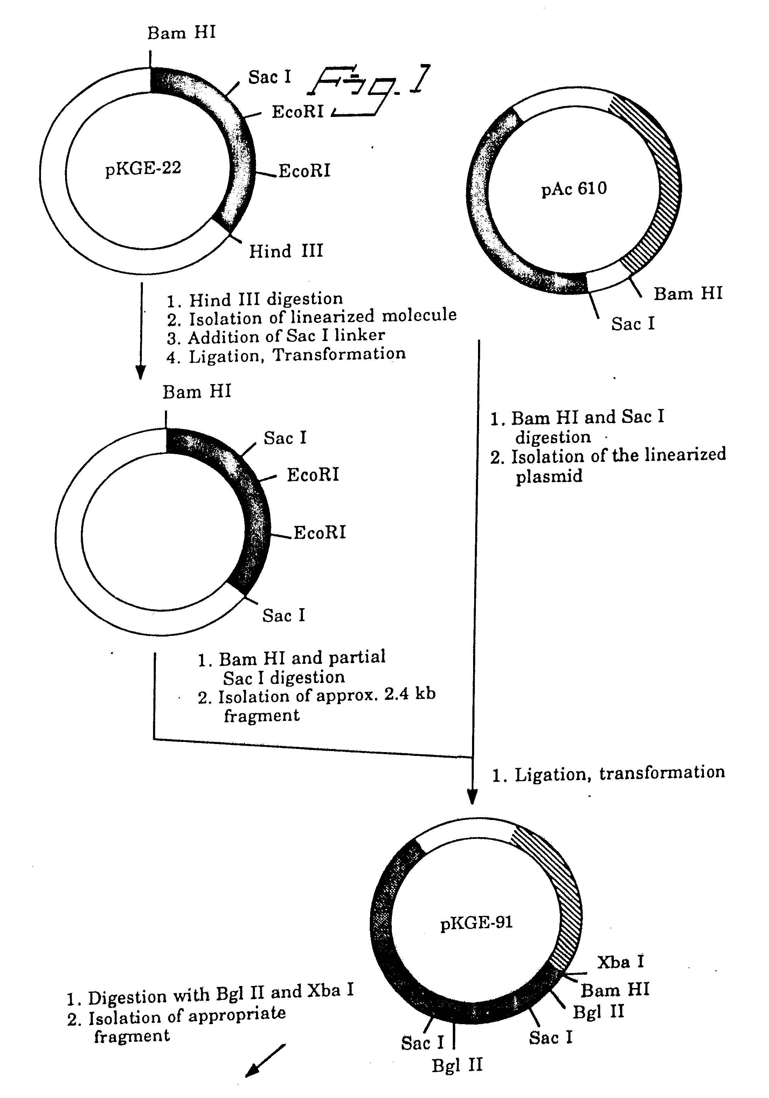

The fragment containing the polyhedrin regulatory signals was isolated by low temperature melting agarose electrophoresis, and the fragment were mixed and ligated with the tPA encoding fragment. The resulting transfer vector pKGE-91 contains 2350 bp tPA cDNA downstream of the polyhedrin promoter. mRNA processing signals were provided in the transfer vector. Fig. 1.

The plasmid pKGE-91 was digested with BglII and XbaI, and the fragment containing pUC, polyhydrin and tPA signal peptide sequences was isolated by LGT agarose electrophoresis.

The isolated ZZ fragment with BamHI and EcoRI ends, the cecropin A encoding sequence on a EcoRI and XbaI fragment and the tPA signal peptide sequence containing pAc610 were ligated and E.coli strain JM83 was transformed. The resulting plasmid was designated pKGE-132.

The methods of Smith and Summers (1987) were followed for selection of recombinant virus. S.frugiperda cells were co-transfected with the transfer plasmid pKGE-132 (2μg) and virus DNA, prepared as total DNA (10μg) from infected cells using the calcium phosphate method. After 6 days the virus containing medium was tested with a plaque assay.

Recombinant virus was detected by hybridization. In brief, the plates were dried for 1 h at 37°C. Colony/Plaque Screen filters (NEN) were lifted from the side of the agar overlay that had been in contact with the cells. Filters were processed according to the manufacturer's protocol and probed with an appropriate fragment labelled by the multiprime reaction according to the suppliers instruction (Amersham).

## EXAMPLE 2

Synthesis of the fusion protein, ZZ-cecropin A

Spodoptera frugiperda Sf9-cells were grown in Hinks medium (Hink 1970), supplemented with 10% FCS either in monolayer culture or in spinner bottles at a cell density of $2x10^6$ per ml (Summers, M.D. and Smith, G.E.: A manual of methods for baculovirus vectors and insect cell culture procedures. Tex.Agric.Exp.Stn.Bull. (1987) No. 1555). Recombinant virus (10 pfu per cell) obtained as described in Example 1 was used to infect the cells. Three days post infection the supernatant was collected by centrifugation. If the ZZ fusion protein was to be affinity purified according to Example 3, the medium was replaced by Hink's medium without FCS.

## EXAMPLE 3

Affinity purification of ZZ-cecropin A

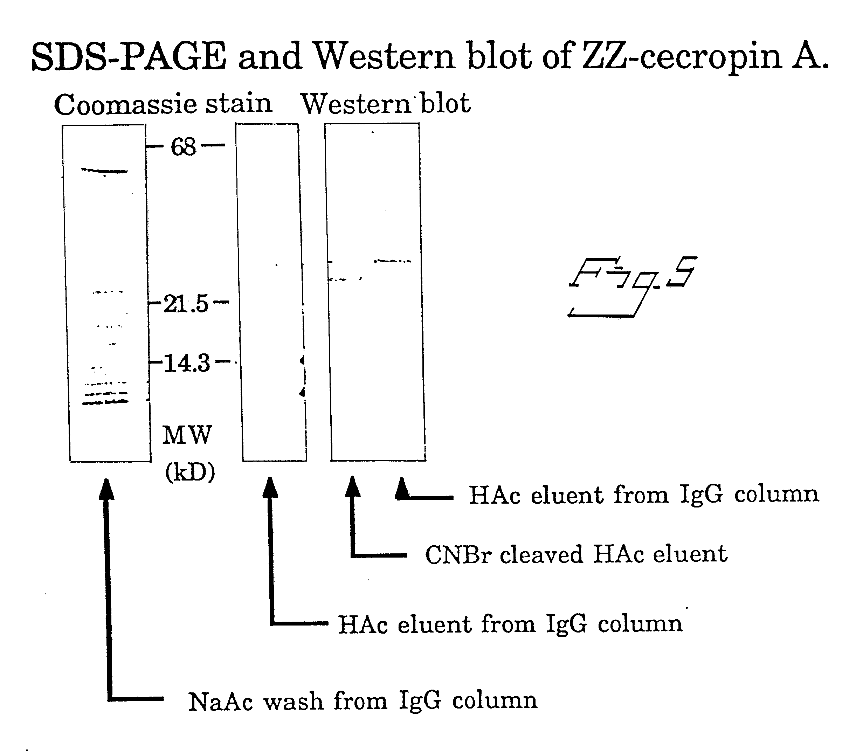

Cell supernatant (350 ml) was passed through a IgG-Sepharose affinity column (3x1cm) that had been equilibrated with buffer (2.5 mM Tris, 7.5 mM NaCl and 0.0025% Tween 20). After washing with ammonium acetate (10mM) the column was eluted with acetic acid (0.2 M). Following removal of the acetic acid by freeze drying the sample was subjected to SDS polyacrylamide gel electrophoresis (Laemmli 1970). The sample shows only a few bands upon staining for protein (Fig. 5). After blotting to nitrocellulose filters, staining with rabbit peroxidase antiperoxidase antibodies and diaminobenzidine, the major protein band could be visualized, showing the presence of the ZZ-part of protein A in this band.

## EXAMPLE 4

Cyanogen bromide (CNBr) cleavage of fusion protein

Affinity purified ZZ-cecropin A was incubated over night with cyanogen bromide (10 mg per mg of protein) in 70% formic acid. The CNBr was removed by freeze drying, and the residue dissolved in distilled water. In Fig. 5 is shown that in the cleaved sample the ZZ-containing band has an apparent molecular weight that is 4kD lower than the ZZ-containing band in the untreated material; a difference indicating the loss of the cecropin A part of the fusion protein.

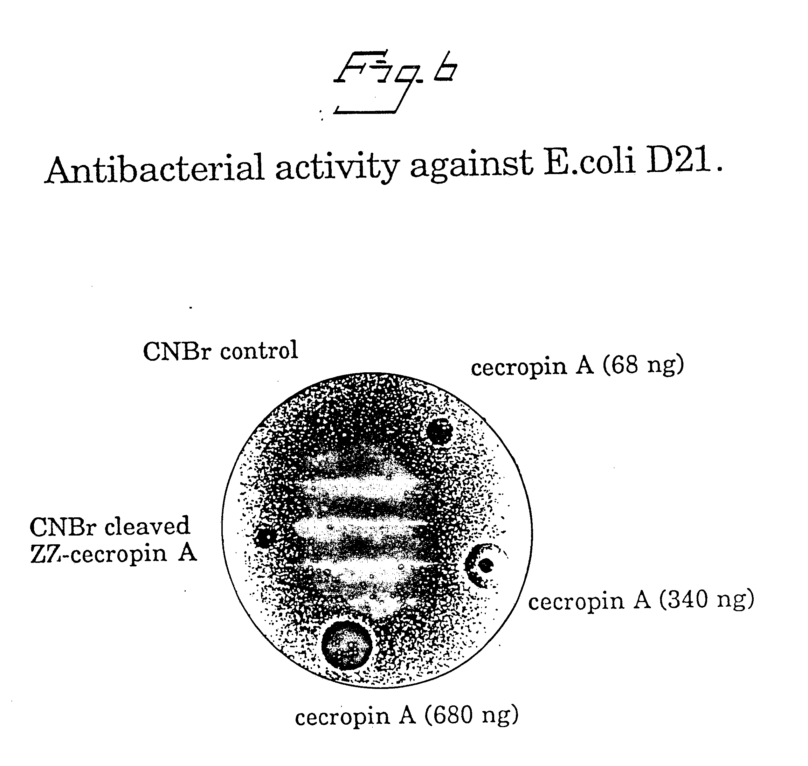

A sample (2µl) was applied to holes in an agarose plate seeded with E.coli, D21, bacteria. After overnight incubation areas without bacterial growth could be seen around the holes, indicating the presence of biologically active cecropin A, (Figure 6).

## EXAMPLE 5

Protein properties

In order to establish the authentic structure of the baculovirus produced cecropin A it was tested with acidic gel electrophoresis. By overlaying the gel with E.coli D21 bacteria a highly specific assay with high resolving power regarding charge differences is obtained (Hultmark et al. 1982).

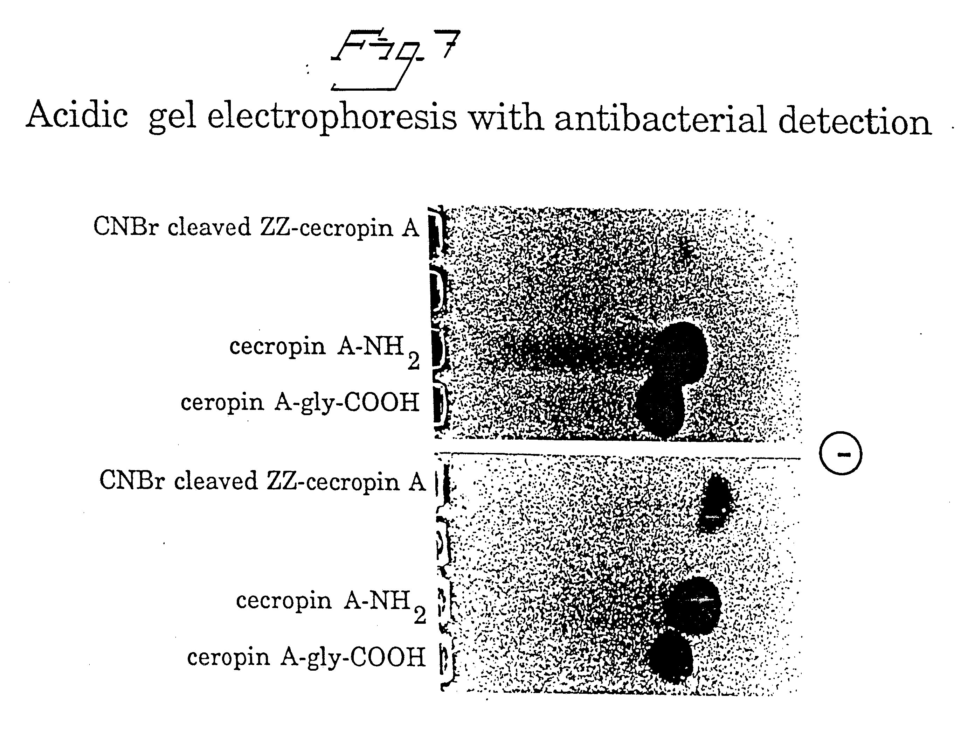

Synthetic preprocecropin A with a glycyl C-terminal extension and natural cecropin A were used as reference proteins. The baculovirus produced protein migrates as natural cecropin A and it is concluded that the recombinant protein is identical to natural cecropin A and thus C-terminally amidated (Figure 7).

## REFERENCES

Edlund et al.: Proc Natl Acad Sci USA 80 (1983) 349-352
Elmblad et al.: Nucl Acid Res 10 (1982) 3291-3301
Hedén et al.: FEBS Lett 194 (1986) 327-332
Hink: Nature 226 (1970) 466-467
Hultmark et al.: 112 Eur.J.Biochem (1982) 207-217
Kleid et al. : Science 214 (1981) 1125-1129
Laemmli: Nature 227 (1970) 680-685
Luckow and Summers: Biotechnology 6 (1988) 47-55
Löwenadler et al.: GENE 58 (1987) 87-97
Maniatis T., Fritsh E.F. and Sambrook J.: Molecular cloning: A laboratory manual. Cold Spring Harbor Laboratory (1982)
Messing J.: Methods Enzymology 101 (1983) 20-78
Okayama H. and Berg P.: Mol Cell Biol 2 (1982) 161-170
Sanger et al.: Proc Natl Acad Sci USA 74 (1977) 5463-5467
Summers M.D. and Smith G.E.: Tex.Agric.Exp Stn.Bull. (1987) No 1555.

**Claims**

1. A method for the production and isolation of a fusion protein, comprising the steps:

    a) constructing a recombinant vector comprising a first DNA sequence coding for a desired peptide or protein and operatively linked to a second DNA sequence coding for an amino acid sequence capable of selective binding to a carrier material, said first or second DNA sequence being preceded by and being operatively linked to a third DNA sequence coding for a eucaryotic signal peptide;

    b) transforming a compatible eucaryotic host with the recombinant vector resulting from step a) such that the combined DNA sequences coding for said fusion protein can be expressed and secreted by said host;

    c) culturing said host to produce said fusion protein;

    d) isolating the fusion protein resulting from step c) by adsorption to a carrier material; and

e) desorbing said fusion protein from said carrier or removing by cleavage said peptide or protein from said amino acid sequence adsorbed on said carrier; and

f) recovering said fusion protein or said peptide or protein.

2. A method according to claim 1, wherein said amino acid sequence is immunogenic.

3. A method according to claim 1 or 2, wherein said amino acid sequence is selected from sequences capable of binding to the constant regions of immunoglobulins.

4. A method according to any preceding claim, wherein said host is selected from insect and mammalian cells.

5. A fusion protein prepared by the method of any preceding claim.

6. A recombinant nucleotide sequence comprising a DNA sequence coding for a desired peptide or protein and operatively linked to a second DNA sequence coding for an amino acid sequence capable of selective binding to a carrier mate rial, said first or second DNA sequence being proceeded by and being operatively linked to a third DNA sequence coding for a eucaryotic signal peptide.

7. A recombinant nucleotide sequence according to claim 6, wherein said second DNA sequence codes for an immunogenic amino acid sequence.

8. A recombinant nucleotide sequence according to claim 6 or 7, wherein said amino acid sequence is selected from sequences capable of binding to the constant regions of immunoglobulins.

9. A recombinant vector, comprising a first DNA sequence coding for a desired peptide or protein and operatively linked to a second DNA sequence coding for an amino acid sequence capable of selective binding to a carrier material, said first or second DNA sequence being proceeded by and being operatively linked to a third DNA sequence coding for a eucaryotic signal peptide.

10. A recombinant vector according to claim 9, wherein said second DNA sequence codes for an immunogenic amino acid sequence.

11. A recombinant vector according to claim 9 or 10, wherein said amino acid sequence is selected from sequences capable of binding to the constant regions of immunoglobulins.

12. A eucaryotic cell harbouring the recombinant nucleotide sequence according to any of claims 6 to 8.

13. A eucaryotic cell harbouring the recombinant vector according to any of claims 9 to 11.

14. A eucaryotic cell according to claim 12 or 13 which is selected from insect and mammalian cells.

15. A process for preparing polyclonal antibodies, comprising immunizing an animal using the fused protein of claim 5, bleeding the animal and recovering the generated antibodies from the animal serum.

16. A process according to claim 15, wherein said animal is a mammal, such as a rabbit or mouse.

17. The use of the fusion protein of claim 5 as a vaccine.

18. The use of the fusion protein of claim 5 for the manufacture of monoclonal antibodies.

Fig. 1

*Fig. 2*

A)

```
BamH I                                              Eco RI
     |----+--------+-----------+---------+------|
     |    |    Z         |         Z        |   |
     |----+--------+-----------+---------+------|
          |                    |              |
        Acc I                Acc I          Acc I
```

B)

```
B                                         A
a                                         c
m                                         c
H                                         I
I
GGATCCCGGCAACACGATGAAGCCGTAGACAACAAATTCAACAAAGAACAACAAAACGCG
---------+---------+---------+---------+---------+---------+
CCTAGGGCCGTTGTGCTACTTCGGCATCTGTTGTTTAAGTTGTTTCTTGTTGTTTTGCGC
GlySerArgGlnHisAspGluAlaValAspAsnLysPheAsnLysGluGlnGlnAsnAla

TTCTATGAGATCTTACATTTACCTAACTTAAACGAAGAACAACGAAACGCCTTCATCCAA
---------+---------+---------+---------+---------+---------+
AAGATACTCTAGAATGTAAATGGATTGAATTTGCTTCTTGTTGCTTTGCGGAAGTAGGTT
PheTyrGluIleLeuHisLeuProAsnLeuAsnGluGluGlnArgAsnAlaPheIleGln

AGTTTAAAAGATGACCCAAGCCAAAGCGCTAACCTTTTAGCAGAAGCTAAAAAGCTAAAT
---------+---------+---------+---------+---------+---------+
TCAAATTTTCTACTGGGTTCGGTTTCGCGATTGGAAAATCGTCTTCGATTTTTCGATTTA
SerLeuLysAspAspProSerGlnSerAlaAsnLeuLeuAlaGluAlaLysLysLeuAsn
```

```
                      A
                      c
                      c
                      I
GATGCTCAGGCGCCGAAAGTAGACAACAAATTCAACAAAGAACAACAAAACGCGTTCTAT
---------+---------+---------+---------+---------+---------+
CTACGAGTCCGCGGCTTTCATCTGTTGTTTAAGTTGTTTCTTGTTGTTTTGCGCAAGATA
AspAlaGlnAlaProLysValAspAsnLysPheAsnLysGluGlnGlnAsnAlaPheTyr

GAGATCTTACATTTACCTAACTTAAACGAAGAACAACGAAACGCCTTCATCCAAAGTTTA
---------+---------+---------+---------+---------+---------+
CTCTAGAATGTAAATGGATTGAATTTGCTTCTTGTTGCTTTGCGGAAGTAGGTTTCAAAT
GluIleLeuHisLeuProAsnLeuAsnGluGluGlnArgAsnAlaPheIleGlnSerLeu

AAAGATGACCCAAGCCAAAGCGCTAACCTTTTAGCAGAAGCTAAAAAGCTAAATGATGCT
---------+---------+---------+---------+---------+---------+
TTTCTACTGGGTTCGGTTTCGCGATTGGAAAATCGTCTTCGATTTTTCGATTTACTACGA
LysAspAspProSerGlnSerAlaAsnLeuLeuAlaGluAlaLysLysLeuAsnAspAla
```

```
                 E
        A        c
        c        o
        c        R
        I        I
CAGGCGCCGAAAGTAGACGCGAATTC
---------+---------+------
GTCCGCGGCTTTCATCTGCGCTTAAG
GlnAlaProLysValAspAlaAsn
```

$$Fig.3$$

```
E
c
o
R
I
CGAATTCTATGAAATGGAAACTGTTCAAGAAAATCGAAAAAGTTGGCCAGAACATCCGTG
---------+---------+---------+---------+---------+---------+
GCTTAAGATACTTTACCTTTGACAAGTTCTTTTAGCTTTTTCAACCGGTCTTGTAGGCAC

    AsnSerMetLysTrpLysLeuPheLysLysIleGluLysValGlyGlnAsnIleArgAsp

ACGGTATCATCAAAGCGGGCCCGGCTGTTGCTGTTGTAGGTCAGGCTACTCAGATCGCCA
---------+---------+---------+---------+---------+---------+
TGCCATAGTAGTTTCGCCCGGGCCGACAACGACAACATCCAGTCCGATGAGTCTAGCGGT

    GlyIleIleLysAlaGlyProAlaValAlaValValGlyGlnAlaThrGlnIleAlaLys

        B
        a           X
        m           b
        H           a
        I           I
AGGGTTAATAGGATCCTCTAGA
---------+---------+--
TCCCAATTATCCTAGGAGATCT

    GlyEndEndAspProLeuGlu
```

Fig. 4

Xba I
Sac I
Bgl II
P

BamH I
Eco RI
ZZ

Eco RI
BamH I
Xba I
cecropin A

Ligation and transformation

Eco RI
Sac I
Xba I

P
Transcriptional
stop signals

pKGE-132

Homologous recombination

Polyhedrin

130 kbp AcNPV genome

# SDS-PAGE and Western blot of ZZ-cecropin A.

Coomassie stain    Western blot

MW
(kD)

*Fig.5*

HAc eluent from IgG column

CNBr cleaved HAc eluent

HAc eluent from IgG column

NaAc wash from IgG column

EP 0 356 409 A2

# Fig. 6

## Antibacterial activity against E.coli D21.

CNBr control

cecropin A (68 ng)

CNBr cleaved
ZZ-cecropin A

cecropin A (340 ng)

cecropin A (680 ng)

EP 0 356 409 A2

## Fig. 7

### Acidic gel electrophoresis with antibacterial detection

CNBr cleaved ZZ-cecropin A

cecropin A-NH$_2$

ceropin A-gly-COOH

CNBr cleaved ZZ-cecropin A

cecropin A-NH$_2$

ceropin A-gly-COOH

EP 0 356 409 A2